# EUROPEAN PATENT SPECIFICATION

(11) **EP 0 411 146 B1**
(45) Date of publication and mention of the grant of the patent: **03.04.1996**
(21) Application number: 90902704.7
(22) Date of filing: 06.02.1990
(51) Int. Cl.: A61N 1/30

(54) **INTERFACE FOR IONTOPHORESIS**
SCHNITTSTELLE FÜR IONTOPHORESE
INTERFACE POUR IONTOPHORESE

(30) Priority: 06.02.1989 JP 25842/89; 06.02.1989 JP 25843/89; 31.08.1989 JP 223166/89; 08.09.1989 JP 231497/89
(43) Date of publication of application: 06.02.1991
(73) Proprietor: HISAMITSU PHARMACEUTICAL CO. INC., Tosu-shi Saga 841 (JP)
(72) Inventor: OKABE, Keiichirou, Tokyo 157 (JP)
(74) Representative: Adams, William Gordon
(86) International application number: JP9000144
(87) International publication number: WO9008571

(56) References cited:
- EP-A- 0 138 347
- EP-A- 0 249 343
- EP-A- 0 269 246
- GB-A- 2 174 605
- JP-A-62 268 569
- JP-A-63 102 768
- US-A- 4 722 726
- US-A- 4 731 049

## Description

The present invention relates to an interface (skin or mucosa member) for iontophorese.

The structure of an interface for iontophorese (iontophoresis) comprises a combination of a reservoir for holding a drug solution and electrodes for current dispersion.

The structure of the reservoir must be such that it is ensured that a predetermined amount of a drug solution reaches the skin interface of a living body within a certain time. The reservoir itself, however, is steric and contains water as the medium, and thus a dilution of the drug occurs. Accordingly, although a more satisfactory structure is required, such a structure has not yet been proposed.

Accordingly, the present invention seeks to provide an interface having a structure suitable for iontophoresis; namely, capable of correctly and safely administering a drug.

Furthermore, the present invention seeks to provide an interface having a structure suitable for iontophoresis; namely, capable of efficiently administering a fine dosage drug such as peptides by maintaining a local high concentration thereof.

Some iontophorese interfaces are known, but EP-A-269246 and US-A-4731049 do not disclose the specific composition and thickness of the membrane used in the present invention.

Therefore, in accordance with the present invention, there is provided an interface for iontophorese comprising, from the top, (i) an electrode, (ii) a reservoir layer for supplementing water, and (iii) a drug layer composed of a rigid porous material or gel material, characterised in that a semipermeable membrane is interposed between the reservoir layer and the drug layer for allowing the flow of water to the drug layer but for preventing the drug contained in the drug layer from penetrating the reservoir layer, wherein said semipermeable membrane is a semipermeable cellulose acetate, polyvinyl chloride or regenerated cellulose semipermeable membrane having a thickness of 0.01 to 100 µm.

Preferably in one embodiment the semipermeable membrane is optionally an ion permselective membrane and has the drug layer plastered on, or attached to, the surface thereof opposite to the reservoir layer and arranged to be brought into contact with a living body.

In the accompanying drawings:-
Figure 1 is a drawing illustrating one embodiment of the interface for iontophorese according to the present invention;
Figure 2 is a drawing illustrating another embodiment of the interface for iontophorese according to the present invention; and,
Figure 3 is a drawing illustrating a further embodiment of the interface for iontophorese according to the present invention.

The interface for iontophorese according to the first embodiment of the present invention comprises a reservoir layer (or water-retaining layer) for supplementing water, a drug layer composed of a rigid porous material or a gel material, and a semipermeable membrane interposed between the reservoir layer for supplementing water and the drug layer. The semipermeable membrane is placed between the drug layer and the reservoir layer for supplementing water to allow a flow of water from a water supplementing reservoir layer to a drug layer, but to maintain a high drug concentration, also functions to prevent a penetration of the drug and miscellaneous microorganisms from the drug layer into the water supplementing reservoir layer.

The reservoir layer or the water-retaining portion for supplementing water to be used in the first embodiment of the present invention may have a vessel structure, or be impregnated water storable fibres, such as cotton, PVA sponge, and cellulose triacetate, or swollen gels holding water. Also, if necessary, it is possible to provide a structure which prevents evaporation to the outside by fixing a cup to the rigid resin surround. Although there are no specific limitations of the thickness of the reservoir layer for supplementing water, preferably the thickness is approximately 0.1 - 5 mm.

Examples of the rigid porous material constituting the drug layer according to the first embodiment of the present invention are porous materials made of ceramics such as bisque, alumina, and zirconia, or synthetic resin materials. The average pore size is preferably several µm to several hundred µm, more preferably 0.1 - 10 µm, and the porosity is preferably about 30 to 90%. Both the pore size and porosity may be selected in accordance with the number of sweat glands of the skin to be treated and the dose of the drug, and are not particularly limited.

Also, ceramics materials and synthetic resin materials, formed into capillary structures by laser working, can be used. The thickness of these materials is not particularly limited, but is preferably about 0.1 mm to 10 mm.

These interface forming means to be used preferably is composed of rigid materials, but in some cases (i.e., deformable when capillaries are used) may be flexible films or sheet materials.

The gel materials forming the drug layer according to the first embodiment of the present invention are hydrophilic resin or polymeric compounds, and examples of the hydrophilic resins include vinyl resins such as polyacrylamide, polyacrylic acid or alkali metal salts thereof or acrylic resins such as esters thereof, polyvinyl pyrrolidone, polyvinyl alcohol, polyvinyl ethyl ether and copolymers thereof, and natural polysaccharides such as gum tragacanth and karaya gum. Examples of the polymeric compounds include methyl cellulose, hydroxypropyl cellulose, hydroxypropylmethyl cellulose, hyaluronic acid or alkali metal salts thereof. Although there are no specific limitations of the thickness of the gel materials, preferably the thickness is approximately 0.01 to 2 mm.

In the preparation of the drug-containing layer by using these materials, the materials and a drug solution are kneaded together. During this operation, water, polyethylene glycol, propylene glycol, and glycerine are used as a flexible plasticizing agent, and as an electrolyte for imparting electroconductivity, sodium chloride, sodium carbonate, phosphoric acid, and sodium citrate, may be formulated therein. As the liquid for dissolving the drug, preferably a buffer constituted of phosphoric acid or citric acid is employed, but depending on the physical properties of the drug, preferably an aqueous mineral acid solution of, for example, hydrochloric acid, or an aqueous citric acid solution, are also used either alone or in a combination thereof.

The semipermeable membrane to be used in the first embodiment of the present invention is of semipermeable cellulose acetate, polyvinyl chloride, or regenerated cellulose and the preferable thickness of the membrane is 0.1 to 100 µm.

The water usable in the present invention is not limited to pure water, and an electrolyte solution such as an aqueous sodium chloride solution may be used instead.

According to the second embodiment of the present invention, there is provided an interface having a structure suitable for iontophoresis; namely, capable of efficiently administering a fine amount dosage drug such as peptides by maintaining a local high concentration thereof.

That is, a peptide type drug such as calcitonin, insulin, and a growth hormone is particularly effective at a fine amount dosage, and to ensure an effective administration of this drug percutaneously or through mucosa by iontophoresis, the concentration thereof must be maintained at a high level.

The present invention has effectively solved this problem by proposing an interface having a drug attached to or plastered on, by, for example, spray drying or spraying, the surface of a drug impermeable and water permeable ion permselective membrane in contact with a living body.

During therapy, water (electrolyte, buffer, etc.) from the water holding portion dissolves the drug attached to the outer surface of a semipermeable membrane or an ion permselective membrane as a solid shape such as powder, through the semipermeable membrane or an ion permselective membrane at the interface thereof with the skin, and maintains a local high concentration thereof for a long time.

As one embodiment of the mode of plastering or attaching the drug according to the present invention, a bound product of an appropriate water-soluble polymer and a drug, i.e., a drug-containing water-soluble layer, can be exemplified.

That is, the drug-containing water-soluble layer is formed by a water-soluble holding or attaching layer containing a predetermined drug. As the water-soluble polymer, any desired water-soluble polymer such as soluble starch (wafer), sodium polyacrylate, and polyvinyl alcohol can be used, and the layer may be formed into a thin film. The extent of the water solubility can be controlled in accordance with the purpose of use.

In the second embodiment of the present invention, the reservoir layer for supplementing water or the water-retaining portion may be exemplified by a vessel structure or a layered structure, i.e., a layer comprising water permeable fibers such as cotton, PVA sponge, and cellulose triacetate, or a swollen gel and a porous ceramics material holding water. Also, if necessary, it is possible to provide a structure which prevents evaporation to the outside by applying a cup to surround of a rigid resin, or a structure in which a solution is introduced only during use.

As the semipermeable membrane, a semipermeable cellulose acetate, polyvinyl chloride, or regenerated cellulose is used, preferably at a thickness of 0.1 to 100 µm.

As the ion permselective membrane, membranes for electrodialysis such as ion exchange membranes composed of a styrene polymer membrane, various fluorinated membranes such as a Nafion^{R} membrane (e.g., fluorinated sulfonic acid membrane, fluorinated carboxylic acid membrane), can be effectively used, and the polarity thereof may be selected in accordance with the polarity of the drug, which is not to be diluted. For example, "Aciplex K-101, A-101" (Asahi Kasei K.K.) can be used. Preferably, the thickness thereof is 0.1 to 0.3 mm.

The drugs contained in the drug layer 3, or adhered on or attached to the semipermeable or ion permselective membrane, are not limited by the molecular weight and other various amounts, and the interface of the present invention is particularly useful for peptide type drugs such as insulin, which can efficiently maintain as high a concentration as possible or requires the presence of sufficient water to promote the efficiency of the iontophoresis, regardless of the fine dosage thereof.

### Antitussive expectorant

sodium chromoglycate, ketotiphen fumarate

### Bronchial vasodilator

formoterol fumarate

### Analgesisic

nalbufin hydrochloride, pentazocin lactate, dichlophenac sodium

### Cardiac

dopamine hydrochloride

### Psychoneurotic stabilizer

perfenadin, phnothiazine

### Antibiotic

cefotetan disodium, dibecacin sulfate, amicacin sulfate, netylmycin sulfate, cisomycin sulfate

### Anti-malignant tumor agent

adriamycin, mytomycin C, bleomycin hydrochloride, rentinan, picivanil, bincrystin sulfate, cisplatin

### Circulatoratory function ameliorator

nicametate citrate, mecrophenoxate hydrochloride, lislidmaleate, calcium hopantenate

### Gout therapeutic agent

aloprinol

### Other peptides

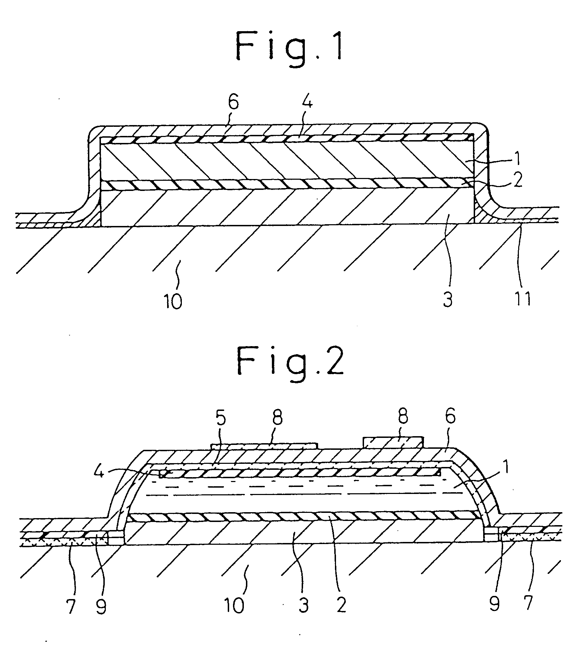

LHRH, enchephalin, endorphin, interferon, insulin, calcitonin, TRH, oxytosin, repressin, vasopressin, glucogan, pituitous hormones (HGH, HMG, HCG, desmopressin acetate), follicle luteinizing hormone
The first embodiment of the present invention will now be explained in detail with reference to Figs. 1 and 2.

In Fig. 1, 1 is a reservoir layer for supplementing water, and can comprise a porous material impregnated with water, an electrolyte or a swollen gel, as described above; 2 is a semipermeable membrane; and 3 is a rigid porous material containing a drug. The reservoir layer 1 for supplementing water, the semipermeable membrane 2, and the rigid porous member 3 are laminated as shown in the Figure, and an electrode 4 comprises an electroconductive rubber, an electroconductive polymer, a carbon film, an aluminum foil, and other metal foils laminated on the upper surface of the reservoir layer 1 for supplementing water. This laminated structure is covered with a flexible supporting member 6, for supporting and fixing same.

The supporting member 6 extends to the living body skin surface 10, and various plastering agents and adhesives 11 are provided at the contact surface thereof with the living skin surface 10.

Another embodiment is shown in Fig. 2. The structure shown in Fig. 2 is equipped with a pair of electrodes and a power supply unit.

The reservoir layer 1 for supplementing water is formed as a closed space, by providing a rigid cup member 5, a laminate of a semipermeable membrane 2 and a rigid porous material 3 mounted at the opening of the cup member 5, and contains an electrolyte or water injected into the closed space. Further, the surface of the cup member 5 is covered with a flexible supporting member 6, and an electrode 4 is mounted on the innerside of the upper surface of the cup member 5.

The supporting member 6 extends to the living body skin surface 10, and an electrode 9 is provided at the contact surface thereof with the living body skin surface, as a counter-electrode of the same material as the electrode 4, and an adhesive electroconductive gel layer 7 (e.g., one to be used as an electrode for the living body) is fixed by plastering onto the surface of the electrode 9 as a counter-electrode.

A power supply unit 8 for supplying an electrical power, and equipped with a battery and an IC, is mounted on the upper surface of the cup member 5. The power supply unit 8, the electrode 4, and the electrode 9 as a counter-electrode are connected by an electroconductive wire (the electroconductive wire is not shown).

The injection inlet for injecting water into the cup member 5 preferably has an opening and closing mechanism (e.g., as illustrated in Japanese Unexamined Patent Publication (Kokai) No. 49-77479).

When the embodiment shown in Fig. 2 is used, if an electroconductive gel layer 7 is plastered onto the living body skin surface 10, the rigid porous layer 3 is brought into close contact with the living body skin surface 10, whereby a closed circuit is obtained, through the living skin tissue, between the electrode 4 and the electrode 9 as a counter-electrode, to thereby complete the preparations for a drug administration.

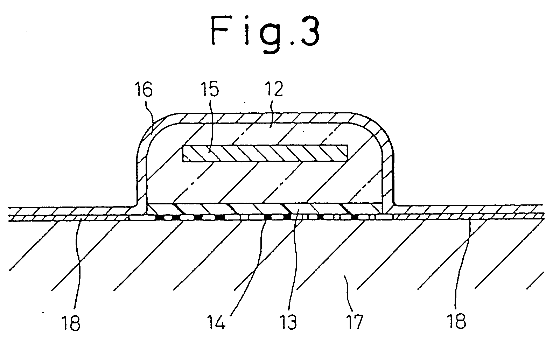

Next, the example according to the second embodiment of the present invention will be explained with reference to Fig. 3.

In Fig. 3, 12 is a reservoir layer for supplementing water and comprises a porous material impregnated with water or electrolyte, or a swollen gel, as described above, or is only a vessel structure; 13 is a semipermeable membrane (e.g., Spectra-Por 3 or Spectra-Por 6, manufactured by Spectrum Medical Industries Inc), or an ion permselective membrane (e.g., Biodyn A, B, C, manufactured by Pall Co.; Selemion CMV, AMV, etc., manufactured by Asahi Glass K.K.), and 14 is a drug arranged on one surface thereof. The inner surface side of the semipermeable or ion permselective membrane 13 is provided with a porous or mesh shaped electrode 15 comprising an electroconductive rubber, an electroconductive polymer, a carbon film, an aluminum foil, or other metal foils. These structures as a whole are covered with a flexible supporting member 16, for supporting and fixing same.

The supporting member 16 further extends to the living body skin surface 17, and various plastering agents and adhesives 18 are provided at the contact surface thereof with the living skin surface 17.

### INDUSTRIAL APPLICABILITY

As mentioned in detail above, the first embodiment of the present invention, through the intermediary of a semipermeable membrane, can supplement an appropriate amount of water without a dilution of the drug solution contained in the drug layer 3 composed of the rigid porous material or gel material, and further, can inhibit the penetration of bacteria from the living body surface or the outside into the water supplementing layer, and thus can carry out an accurate administration over a long term.

On the other hand, the second embodiment of the present invention, through the intermediary of a semipermeable membrane or an ion permselective membrane, can supplement appropriate amounts of water without diluting the drug held on the outer surface thereof, and can inhibit the penetration of bacteria from the living body surface or outside into the water holding portion, and thus can perform an efficient and correct administration over a long term.

Therefore, according to the present invention, the above-mentioned various drugs can be effectively administered to the living bodies, percutaneously or through mucosa.

## Claims

1. An interface for iontophorese comprising, from the top, (i) an electrode (4, 15), (ii) a reservoir layer (1, 12) for supplementing water, and (iii) a drug layer (3, 14) composed of a rigid porous material or a gel material, characterised in that a semipermeable membrane (2, 13) is interposed between the reservoir layer (1, 12) and the drug layer (3, 14) for allowing the flow of water to the drug layer (3, 14) but for preventing the drug contained in the drug layer (3, 14) from penetrating the reservoir layer (1, 12), wherein said semipermeable membrane (2, 13) is a semipermeable cellulose acetate, polyvinyl chloride or regenerated cellulose semipermeable membrane having a thickness of 0.01 to 100 µm.

2. An interface for iontophorese as claimed in claim 1, wherein said rigid porous material is a ceramic porous material or synthetic resin material having an average pore size of 0.01 to 10 µm and a porosity of 30 to 90%.

3. An interface for iontophorese as claimed in claim 1 or claim 2, wherein said gel material is a hydrophilic resin or polymeric compound.

4. An interface for iontophorese as claimed in any one of claims 1 to 3, wherein said semipermeable membrane is optionally an ion permselective membrane (13) and has the drug layer (14) plastered on, or attached to, the surface thereof opposite to the reservoir layer (12) and arranged to be brought into contact with a living body.

5. An interface for iontophoresis as claimed in claim 4, wherein said semipermeable membrane (13) has a thickness of 0.1 to 100 µm.

6. An interface for iontophoresis as claimed in claim 5, wherein said ion permselective membrane (13) is a styrene polymer membrane, fluorinated sulfonic acid membrane or fluorinated carboxylic acid membrane having a thickness of 0.1 to 100 µm.

## Patentansprüche

1. Eine Schnittstelle für die Iontophorese, enthaltend von oben (i) eine Elektrode (4, 15) (ii), eine Reservoirschicht (1, 12) zur Versorgung mit Wasser und (iii) eine Arzneistoffschicht (3, 14), die aus einem festen porösen Material oder einem gelartigen Material zusammengesetzt ist, dadurch gekennzeichnet, daß eine semipermeable Membran (2, 13) zwischen der Reservoirschicht (1, 12) und der Arzneistoffschicht (3, 14) angeordnet ist, um den Fluß des Wassers durch die Arzneistoffschicht (3, 14) zu ermöglichen, aber den in der Arzneistoffschicht (3, 14) enthaltenen Arzneistoff von der Penetration in die Reservoirschicht (1, 12) abzuhalten, wobei die semipermeable Membran (2, 13) ein semipermeables Celluloseazetat, Polyvinylchlorid oder eine semipermeable Membran aus regenerierter Cellulose mit einer Dicke von 0,01 bis 100 µm ist.

2. Eine Schnittstelle für die Iontophorese nach Anspruch 1, worin das feste poröse Material ein keramisches poröses Material oder ein synthetisches Harz mit einer Porengröße von 0,01 bis 10 µm und einer Porosität von 30 bis 90% ist.

3. Eine Schnittstelle für die Iontophorese nach Anspruch 1 oder 2, dadurch gekennzeichnet, daß das gelartige Material ein hydrophiles Harz oder eine polymere Verbindung ist.

4. Eine Schnittstelle für die Iontophorese nach einem der Ansprüche 1 bis 3, worin diese semipermeable Membran gegebenenfalls eine permselektive Membran (13) ist und eine Arzneistoffschicht (14) auf deren Oberfläche gegenüber von der Reservoirschicht (12) aufgeklebt oder daran befestigt aufweist und angeordnet ist, um in Kontakt mit einem lebenden Körper gebracht zu werden.

5. Eine Schnittstelle für die Iontophorese nach Anspruch 4, worin die semipermeable Membran (13) eine Dicke von 0,1 bis 100 µm aufweist.

6. Eine Schnittstelle für die Iontophorese nach Anspruch 5, worin die permselektive Membran (13) eine Styrolpolymer-Membran, eine fluorierte Sulfonsäure-Membran oder fluorierte Carbonsäure-Membran einer Dicke von 0,1 bis 100 µm ist.

## Revendications

1. Interface pour ionophorèse, comprenant, depuis la partie supérieure, (i) une électrode (4, 15), (ii) une couche réservoir (1, 12) pour un apport d'eau, et (iii) une couche médicamenteuse (3, 14) constituée d'une matière poreuse rigide ou d'une matière sous forme de gel, caractérisée en ce qu'une membrane semiperméable (2, 13) est interposée entre la couche réservoir (1, 12) et la couche médicamenteuse (3, 14) pour permettre l'écoulement d'eau à la couche médicamenteuse (3, 14), mais pour empêcher le médicament présent dans la couche médicamenteuse (3, 14) de pénétrer dans la couche réservoir (1, 12), ladite membrane semiperméable (2, 13) étant une membrane semiperméable d'acétate de cellulose, d'un polymère de chlorure de vinyle ou de cellulose régénérée ayant une épaisseur de 0,01 à 100 µm.

2. Interface pour ionophorèse suivant la revendication 1, dans laquelle la matière poreuse rigide est une matière poreuse céramique ou une résine synthétique ayant un diamètre moyen des pores de 0,01 à 10 µm et une porosité de 30 à 90 %.

3. Interface pour ionophorèse suivant la revendication 1 ou la revendication 2, dans laquelle la matière sous forme de gel est une résine ou un composé polymérique hydrophile.

4. Interface pour ionophorèse suivant l'une quelconque des revendications 1 à 3, dans laquelle la membrane semiperméable est de manière facultative une membrane sélectivement perméable aux ions (13) et comprend la couche médicamenteuse (14) collée sur, ou fixée à, sa surface opposée à la couche réservoir (12) conçue pour être mise en contact avec un organisme vivant.

5. Interface pour ionophorèse suivant la revendication 4, dans laquelle la membrane semiperméable (13) a une épaisseur de 0,1 à 100 µm.

6. Interface pour ionophorèse suivant la revendication 5, dans laquelle la membrane sélectivement perméable aux ions (13) est une membrane de polymère de styrène, une membrane d'acide sulfonique fluoré ou une membrane d'acide carboxylique fluoré ayant une épaisseur de 0,1 à 100 µm.
